(19) Europäisches Patentamt
European Patent Office
Office européen des brevets

(11) Veröffentlichungsnummer: **0 001 640**
**B1**

(12) # EUROPÄISCHE PATENTSCHRIFT

(45) Veröffentlichungstag der Patentschrift: **24.08.83**

(51) Int. Cl.³: **A 61 B 6/00**, H 05 G 1/46

(21) Anmeldenummer: **78101198.6**

(22) Anmeldetag: **23.10.78**

(54) **Röntgeneinrichtung.**

(30) Priorität: **25.10.77 US 845138**

(43) Veröffentlichungstag der Anmeldung:
**02.05.79 Patentblatt 79/9**

(45) Bekanntmachung des Hinweises auf die Patenterteilung:
**24.08.83 Patentblatt 83/34**

(84) Benannte Vertragsstaaten:
**DE FR**

(56) Entgegenhaltungen:
**DE - A - 2 220 444**
**DE - A - 2 235 252**
**DE - A - 2 323 710**
**DE - A - 2 345 947**
**DE - A - 2 539 898**
**DE - A - 2 647 928**
**DE - A - 2 741 673**
**FR - A - 2 115 423**
**FR - A - 2 220 230**
**FR - A - 2 225 911**
**US - A - 3 917 949**
**US - A - 4 035 648**

(73) Patentinhaber: **KOCH & STERZEL GmbH & Co.**
**Kruppstrasse 82 - 96**
**D-4300 Essen 1 (DE)**

(72) Erfinder: **Hellstrom, Melbourne**
**116 St.Ives Dr.**
**Severena Park (US)**

**EP 0 001 640 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

Röntgeneinrichtung

Die vorliegende Erfindung betrifft eine Röntgeneinrichtung gemäß dem ersten Teil des Anspruchs 1. Eine derartige Röntgeneinrichtung ist aus der US—A—4 035 648 bekannt.

Derartige Einrichtungen sind insbesondere dafür vorgesehen, ein Objekt zu untersuchen, von dem angestrebt wird, eine möglichst qualitativ einwandfreie Abbildung zu erzielen.

In diesem Zusammenhang werden heutzutage in der Röntgentechnik Röntgenröhren benutzt, bei denen meistens zwei Brennfleckgrößen einstellbar sind, und zwar ein kleiner und ein großer. Durch Veränderung des Brennflecks wird die Bildqualität beeinflußt, und zwar ist die Auflösung der Röntgenabbildung umso größer, je kleiner der Brennfleck ist. Eine andere Tatsache zur Beeinflussung der Qualität der Röntgenabbildung, ist die Bewegung des Patienten, die entweder gewollt oder ungewollt abläuft, und die eine Bewegungsunschärfe auf dem Röntgenbild hervorruft. Beispiele für die ungewollten Bewegungen sind: die Atmungsbewegung, die Bewegung des Herzmuskelsystems und die Bewegung des Verdauungssystems.

Um die durch diese Art der Bewegung hervorgerufenen schädlichen Effekte für die Qualität des Röntgenbildes auf ein Minimum zu bringen, wird es angestrebt, die Belichtungszeit so klein wie möglich zu halten. Dies führt jedoch zu Komplikationen in Bezug auf das Leistungsvermögen der Röntgenröhre, die aus den relativ großen Beträgen der abzuleitenden Wärme manifestieren. Meistens ist die elektrische und thermische Kapazität der Drehanode in Form von Röhrenbelastungstabellen bzw. Kurven angegeben, welche von dem Röhrenhersteller mitgeliefert werden.

Im Hinblick auf eine bessere Wärmedichteverteilung auf dem Drehanodenteller ist es bekannt, die Drehanode einer Röntgenröhre mit zwei Geschwindigkeiten, und zwar einer Standardgeschwindigkeit bei niedriger Drehzahl als auch einer Maximal-Geschwindigkeit bei hoher Drehzahl zu betreiben. Um die richtige Belichtung eines Röntgenfilmes zu erreichen werden die Belichtungs-Faktoren im wesentlichen auf Grund von Erfahrungen des Benutzers gewählt.

Der vorliegenden Erfindung liegt die Aufgabe zugrunde, eine Röntgeneinrichtung der eingangs genannten Art zu schaffen, die sich durch einen fast vollständig automatisierten Steuerungsablauf insbesondere im Hinblick auf die Optimierung der Bildqualität und die Maximierung der Lebensdauer der Strahlenquelle auszeichnet.

Erfindungsgemäß wird die Aufgabe durch die im Kennzeichen des Hauptanspruchs festgehaltenen Merkmale gelöst.

Vorzugsweise ist der erfindungsgemäße Gegenstand auf eine Röntgeneinrichtung ausgerichtet, die durch Kontrolle einer Mehrzahl von auswählbaren Prozeßsteuer-Programmen betrieben wird, die in einem Informationsspeicher eines Mikroprozessors gespeichert sind. Ein solches Programm ist zur Optimierung der Bildqualität und zwar in Bezug auf die Auflösung der Röntgenabbildung und die gleichzeitige Maximierung der Lebensdauer der Röntgenröhre vorgesehen.

Wie bekannt, nimmt der Benutzer folgende Eingaben vor: die Eingabe des Röntgenröhrenstromes (mA), der Belichtungszeit (s) und der Röntgenröhrenspannung (kV) bzw. des Röntgenröhrenstrom-Belichtungszeitproduktes und der Röntgenröhrenspannung, die entsprechend dem jeweiligen diagnostischen Verfahren ausgewählt worden sind, oder es erfolgt ein organprogrammierter Abruf, wobei zusätzlich z.B. die Dicke des Patienten eingegeben wird. Ein hierin nachher näher definierter Prozeßsteuerrechner wählt durch ein Prozeßsteuer-Programm ein geeignetes Instruktions-Programm und hierbei mit arbeitende Operationsprogramme derart aus, daß der größte Wert des vom Röntgendiagnostikapparat lieferbaren Röntgenröhrenstromes verwendet wird um den korrespondierenden kleinstmöglichen Wert der Belichtungszeit zu berechnen, und zwar durch Division des gewählten mAs-Produktes durch den maximalen Röntgenröhrenstrom. Nach Berechnung der minimal möglichen Zeit werden die somit ermittelten Werte verglichen mit einem aus einer Anzahl von digital in dem Informationsspeicher (Referenzprogramm) gespeicherten Röntgenröhren-Belastungsdiagrammen. Das zuerst gewünschte Röntgenröhren-Belastungsdiagramm ist zum Zwecke der Bildoptimierung das des kleinsten Brennflecks und der Standardgeschwindigkeit. Auf Grund des Vergleichs der eingegebenen Belichtungsparameter mit dem Belastungsdiagramm, und zwar derart, daß kein Widerspruch hervorgerufen wird, d.h., wenn keine Überschreitung des gewählten Röntgenröhren-Belastungsdiagramms auftritt, leitet der Rechner die notwendigen Schritte zur Ermöglichung der Belichtung ein. Um eine exakte Bestimmung der thermischen Auslastung der Röntgenröhre zu ermitteln, stehen der Prozeßsteuerautomatik in dem Informationsspeicher entsprechend Programme (hierbei später Instruktions- oder Operations-Programme genannt) zur Verfügung. Die Programme erlauben die Überprüfung der vorhandenen Wärmekapazität der Drehanode, die nötigen Wartezeiten zwischen aufeinanderfolgenden Belichtungen bei Serien, und die Speicherung der Historie (Anzahl der erfolgten Belichtungen und ihrer Werte, Betriebszeit, usw.) der Röntgenröhre. Wenn andererseits das Röntgenröhren-Belastungsdiagramm überschritten wird, bewirkt das aufgerufene Programm eine Veränderung der Belichtungsparameter derart, daß eine Abnahme des Röntgenröhrenstromes erfolgt bei gleichzeitiger Konstanthaltung des mAs-Produktes, und zwar durch Zunahme der Belichtungszeit. Die neu errechneten Belichtungsparameter werden wiederum verglichen mit dem ausgewählten Belastungsdiagramm, und zwar um festzustellen, ob die Belichtung nunmehr gewährt werden kann oder nicht.

Wenn die Belichtung noch nicht durch die Prozeßsteuereinheit gewährt werden kann, wird der Prozeß wiederholt bis ein (mAs) Produkt gewährbar erscheint. Der Prozeß der Abnahme des Röntgenröhrenstromes und der Zunahme der Zeit, reduziert die Wärmedichteverteilung, wodurch zwar die Möglichkeit größer wird den Forderungen der Röntgenröhren-Belastungsdiagramme zu entsprechen, aber die Zunahme der Aufnahmezeit jedoch einen schädlichen Effekt bezüglich der Bildauflösung ergeben kann, und zwar jene, die hinsichtlich der Bewegungsunschärfe verursacht werden. Deshalb wird jedesmal ein Wechsel der Belichtungsparameter bei konstantem mAs-Produkt gemacht, wobei die daraus resultierende Belichtungszeit mit einem vorbestimmten Wert verglichen wird, der von dem Benutzer als maximal zulässiger Wert im Hinblick auf die tolerierbare Bewegungsunschärfe eines Organbereiches der Abbildung gewählt wird. Wenn die errechnete Zeit diesen vorbestimmten Wert überschreitet, kann keine Belichtung gewährt werden. Dann wird die Strahlenausbeute erhöht, durch Zunahme der Röntgenröhrenspannung und Abnahme des Röntgenröhrenstromes bei gleicher thermischer Belastung dies jedoch in einer solchen Weise, daß der Filmschwärzungsfaktor der Belichtung konstant bleibt. Eine Veränderung der Röntgenröhrenspannung darf jedoch nur in einem so kleinen Betrag erfolgen, daß die Charakteristik der zu erwartenden Aufnahme nicht zu stark verändert wird.

Wenn die Kombination von konstantem Wechsel des mAs-Produktes und des Röntgenröhrenspannungswechsels nicht dem Block von Faktoren entspricht, der eine Belichtung bei dem zuerst gewünschten Röntgenröhren-Belastungsdiagramm erlaubt, dann geht das Rechnerprogramm zu dem am nächsten wünschenswerten Röntgenröhren-Belastungsdiagramm über, d.h. kleiner Brennfleck und Maximalgeschwindigkeit; worauf das Testverfahren mit den von dem Benutzer eingegebenen Originalfaktoren wiederholt wird. Beim Nichtgewähren einer Belichtung werden beim Programmablauf dann alle möglichen Belastungsdiagramme der möglichen Röntgenröhren-Betriebsarten durchgegangen und in Bezug auf ihr Verhalten, das auf den Forderungen einer optimalen Bildqualität und einer hohen Lebensdauer der Röntgenröhre basiert, und zwar bis entweder ein zulässiger Block von Belichtungsfaktoren gefunden worden ist oder daß keine Belichtung auf Grund der Zwänge der Programms möglich ist. Ein derartiger Fall hätte zur Folge daß die Belichtung solange nicht gewährt wird, bis vom Benutzer andere Belichtungsdaten (mA, s, kV) eingegeben werden.

Zusätzliche Funktionen werden ebenfalls durch entsprechende Programme bewältigt, wie z.B. die Überprüfung der vorhandenen Wärmekapazität der Drehanode, die notwendige Wartezeit zwischen aufeinanderfolgenden Belichtungen bei Tomographieserien, sowie beispielsweise ferner die Speicherung der Historie der Röntgenröhre, die Speicherung der Belichtungsfaktoren und ihr Abruf auf einer automatischen Basis, und die Registrierung der geschätzten Patientendosis.

Anhand der in der Zeichnung dargestellten Beispiele sei die vorliegende Erfindung näher erläutert, und zwar zeigen:

Fig. 1, ein Blockdiagramm, das den wesentlichen Aufbau des erfindungsgemäßen Gegenstandes umreißt,

Fig. 2, ein Blockdiagramm, das die wesentlichen Elemente von einem digitalen Speicherprogramm-Rechner darstellt,

Fig. 3, ein Blockdiagramm, das die wesentlichen Elemente von einem zentralen Verarbeitungsgebilde eines digitalen Rechners umreißt,

Fig. 4, ein Blockdiagramm, das eine vorteilhafte Ausführung des erfindungsgemäßen Gegenstandes darstellt,

Fig. 5 bis 11, Flußdiagramme, die eine Mehrzahl von auswählbaren Instruktionsprogrammen hervorheben, die in dem Informationsspeicher des Rechners zur Steuerung des Systems nach Fig. 4 enthalten sind.

Bevor die Einzelheiten der Erfindung ausführlich diskutiert werden, wird auf folgende hauptsächliche Betrachtungen hingewiesen, die sich auf einen typischen digitalen Rechner beziehen, wie er zuvor erwähnt ist. Typischerweise weist ein digitaler Rechner drei hauptsächliche Elemente auf,

a) eine zentrale Verarbeitungseinheit (ZVE)

b) einen Informationsspeicher

c) eine Mehrzahl von Ein- und Ausgabeeinheiten.

Der Informationsspeicher dient dazu, Instruktionen und Daten zu speichern, wobei die Instruktionen codierte Informationsteile darstellen, die die Aktivitäten der ZVE beeinflussen, und wobei die Daten codierte Informationsteile darstellen, die von der ZVE verarbeitet werden. Eine Gruppe von logisch bezogenen Instruktionen, die in dem Informationsspeicher gespeichert ist, wird als ein Programm bezeichnet. Die ZVE liest demgemäß jede Instruktion des Informationsspeichers in einer logisch bestimmten Reihenfolge und benutzt sie um die Prozeßaktionen anzuregen. Wenn die Instruktionsfolge zusammenhängend und logisch ist, dann produziert das Programm verständliche und gewünschte Resultate.

Wie oben erwähnt, wird der Informationsspeicher benutzt, um die zu manipulierenden Daten ebenso wie die die Manipulation beeinflussenden Instruktionen zu speichern. Die ZVE kann schnell ingendwelche in dem Informationsspeicher gespeicherten Daten zugänglich machen und enthält Zwischenspeicherregister. Desöfteren sind die Daten zur Verarbeitung im voraus nicht bekannt oder können nicht aus existierenden Informationen hergeleitet werden. Dieses Problem wird dadurch gelöst, daß der Rechner mit einer oder mehreren Eingabe- und Ausgabeeinheiten versehen ist. Die ZVE adressiert dann diese Einheiten und bewirkt die Eingabe und Ausgabe der Daten von oder zu externen hiermit gekoppelten Einrichtungen. Eine alternierende Methode der Kommunikation mit externen Einrichtungen ist die, jeder Einrichtung eine einzige Adresse zuzuordnen, wodurch es der ZVE möglich ist, die Einrichtungen als Speicherplätze zu behandeln. Der Ausgang kann z.B. zu einer Anzeige führen, die für den Benutzer vorgesehen ist oder zu einer peripheren Einrichtung, wie z.B. einem Zeilendrucker, einer Speichereinheit oder es können Prozeß-Steuer-Signale gebildet werden, die die Operationen von einem anderen System beeinflussen.

Die der Erfindung zugrundeliegende Aufgabe ist auf eine computerisierte Steuerung einer Röntgeneinrichtung ausgerichtet, und zwar um die von einem Röntgentechniker bei einer radiologischen Untersuchung vorzunehmenden Schritte zu vereinfachen.

Von primärem Interesse ist jedoch, eine bestmögliche Abbildung zu erzielen, und zwar in Bezug auf die Auflösung und gleichzeitiger Minimalisierung der Verwendung der Maximalgeschwindigkeit der Drehanode, da ein solcher Gebrauch zur Reduktion der Lebensdauer der Lager führt.

Drei Ziele ergeben sich aus dieser Anforderung.

Erstens ist es wünschenswert, die Röntgenröhre(n) nach einer Prioritätsbeziehung hinsichtlich des Brennflecks und der Drehanodengeschwindigkeit zu betreiben:

a) kleiner Brennfleck, Standardgeschwindigkeit,
b) kleiner Brennfleck, Maximalgeschwindigkeit,
c) großer Brennfleck, Standardgeschwindigkeit,
d) großer Brennfleck, Maximalgeschwindigkeit.

Diese Bedingungen führen zu ausgesprochen hoher Auflösung der Röntgenbilder.

Zweitens ist hinsichtlich der Belichtungsparameter, und zwar gleichgültig ob sie von dem Benutzer bei der Dreipunkttechnik durch Eingabe der Röntgenröhrenspannung, des Röntgenröhrenstromes und der Beilichtungszeit bei der Zweipunkttechnik durch Eingabe der Röntgenröhrenspannung und des mAs-Produktes oder mit Hilfe der durch organprogrammierten Technik, und zwar in Abhängigkeit von der durchzuführenden Untersuchung als auch von irgendwelchen physikalischen Spezifikationen des Patienten eingestellt werden, noch der Filmschwärzungsfaktor zu beachten, und zwar ist dieser in die Überlegungen bzw. Rechnungen einzubeziehen.

Der Filmschwärzungsfaktor FSF ist proportional zu dem Produkt von Röntgenröhrenstrom (mA), Belichtungszeit (s) und Röntgenröhrenspanning (kV) und stellt sich als Formel wie folgt dar:

$$FSF \sim (mA) \times (s) \times (kV)^n$$

wobei n in der Größenordnung von 4 oder 5 liegt.

Während die Belichtungsfaktoren geändert werden können, ist es im Hinblick auf einen akzeptierbar geschwärzten Film nichtsdestoweniger wünschenswert, den Wert des Filmschwärzungsfaktors vollständig konstant zu halten.

Drittens ist es wünschenswert, die minimal mögliche Aufnahmezeit zu verwenden für einen Block von Betriebsbedingungen, und zwar zwecks Vermeidung der Verwischung.

Es ist jedoch zu bemerken, daß das erste und das dritte der obenerwähnten drei Ziele unvereinbar sind, da für ein gegebenes mAs-Produkt eine kürzere Belichtungszeit in einem höheren Röntgenröhrenstrom resultiert, und dies kann bei irgendeiner feststehenden Röntgenröhrenspannung den Gebrauch des großen Brennflecks erforderlich machen, und zwar auf Grund hoher Leistungsabgabe der Drehanode der Röntgenröhre.

Anhand der Fig. 1 werden nunmehr die wesentlichsten Aspekte des erfindungsgemäßen Gegenstandes erläutert: Wie aus Fig. 1 hervorgeht, ist eine digitale Steuerung 10 einer diagnostischen Röntgeneinrichtung vorgesehen, welche eine Datenverarbeitungseinheit in Form eines digitalen Speicherprogramm-Rechners 12 umfaßt, der geeignet ist, digitale Steuersignale an einen den Röntgengenerator beeinflussenden Kopplungskreis 14 zu liefern, der auf die digitalen Steuersignale derart reagiert, daß analoge Steuersignale hervorgerufen werden, welche zu einem in einem Untersuchungsraum angeordneten Röntgengenerator 16 führen, welcher z.B. einen Hochspannungstransformator mit Gleichrichtereinheit beinhaltet, der mit einer oder mehreren Röntgenröhre(n) verbunden ist. Die Aufteilung zwischen dem Kopplungskreis 14 und dem Röntgengenerator 16 ist ziemlich willkürlich gewählt und wird physikalisch durch die kommerzielle Herstellung und durch technische Gründe bestimmt. Es ist denkbar, z.B., daß die Elemente 14 und 16 in demselben Gehäuse oder in separaten Gehäusen in demselben Raum untergebracht sind. Der Röntgengenerator 16 liefert zusätzlich benötigte Rückkopplungssignale, welche zu dem digitalen Speicherprogramm-Rechner 12 mit Hilfe von Analog-Digital-Wandlern 13 zurückgeführt sind. Weiterhin sind Eingabeeinheiten 20 vorgesehen, die aus Tastenfeldern, Druckknöpfen, Schaltern und anderen Einrichtungen bestehen und zur Eingabe z.B. der Belichtungsparameter, d.h. der Röntgenröhrenspannung des Röntgenröhrenstromes und der Belichtungszeit, ebenso wie der radiologischen Verfahrenswahl (z.B. Tomographie) dienen, und die geeignet sind, die Eingangssignale mit dem digitalen Speicherprogramm-Rechner zu koppeln, und zwar zusammen mit anderen gewünschten Eingangssignalen von unter anderem externen, zusätzlichen digitalen Unterstützungssystemen 22. Solche Unterstützungssysteme können z.B. das hospitale Rechnersystem oder ein Datensystem der radiologischen Abteilung umfassen, welche zum Zwecke der Registration des Patienten der Aufzeichnung usw. vorhanden sind. Zusätzlich ist der Rechner 12 mit geeigneten Anzeige- und Ausgabeeinheiten 24 gekoppelt, die z.B. aus Kathodenstrahlröhren, LED, alphanumerischen Anzeigen, Druckern, Lautsprechern usw. bestehen können, und die notwendig und erwünscht sind für die Informationsübermittlung an den Benutzer oder das Hilfspersonal über die jeweilige Systemoperation.

Der Rechner 12 umfaßt vorzugsweise einen Mikroprozessor, der eine Mehrzahl von Instruktionsprogrammen in einem digitalen Informationsspeicher z.B. einem ROM oder PROM besitzt. Solche Einrichtungen sind allgemein bekannt und handelsüblich. Die Ausgestaltung des Mikroprozessors des Rechners 12 ist ausführlicher dargestellt in Fig. 2 und umfaßt eine integrierte zentrale Verarbeitungseinheit (ZVE) 26, die z.B. aus einer acht bit parallel-gesteuerten Verarbeitungseinheit besteht. Die ZVE 26 ist mit einem Adress-Bus 28, welcher einen sechzehn bit Drei-Zustands-Adress-Bus umfaßt, ebenso wie mit einem acht bit bi-direktionalen Drei-Zustands-Daten-Bus 30 gekoppelt. Zusätzlich ist ein Steuer-Bus 32 vorgesehen, der z.B. geeignet ist, sechs Kontrollausgänge, vier Kontrolleingänge, vier Leistungseingänge und zwei Taktgebereingänge zu manipulieren. Der Rechner 12 enthält zusätzlich zu der ZVE 26 einen digitalen Informationsspeicher 34, der z.B. aus einem oder mehreren PROM's 34A und aus einem oder mehreren RAM's 34B besteht, welche leicht an die ZVE 26 angepaßt werden können. Das PROM 34A ist mit den Adress-Daten und Kontroll-Bussen 28, 30 und 32 gekoppelt und ist geeignet, bis zu 1024 acht bit-Instruktionswörter zu speichern. Das RAM 34B hat eine Kapazität zur Speicherung von 512 Wörtern. Zusätzlich sind ein oder mehrere Ein- und Ausgabeeinheiten 36 mit den Adress-, Daten- und Kontroll-Bussen 28, 30 and 32 gekoppelt.

In. Fig. 3 sind die drei funktionalen Einheiten, die in der ZVE 26 nach Fig. 2 enthalten sind, dargestellt. Wie dargestellt, besteht es aus einer arithmetisch-logischen Einheit (ALE) 38, einer Takt-Steuer-Schaltung 40 und einem RAM 42, bestehend aus einer Anordnung von Speicherregistern. Solch eine Anordnung ist bekannt und schließt normalerweise unter anderem ein akkumulatives Register, einen Programm-Zähler, ein Instruktions-Register und Decoder sowie ein Adress-Register ein.

Die ALE 38 ist das Teil der zentralen Hardware, welches, wie sein Name ausdrückt, die arithmetisch-logischen Operationen dafür auf Grund der binären Datenbasis durchführt. Obgleich es nicht gezeigt ist, enthält das ALE ein Addierglied, welches fähig ist, die Inhalte von zwei Registern in Übereinstimmung mit der Logik der binären Arithmetik zu kombinieren. Diese Maßnahme erlaubt dem Prozessor arithmetische Manipulationen der Daten durchzuführen, welche er von dem Informationsspeicher 34 und seinen anderen Eingängen erhält. Die Takt-Steuer Steuer Schaltung 40 ist die erste Funktionseinheit in der ZVE für die Aufrechter haltung der richtigen Folge der für den gewünschten Verfahrensablauf erforderlichen Vorgänge. Nachdem eine Instruktion eingeholt und decodiert ist, gebraucht die Steuerschaltung die geeigneten Signale zur Anregung der richtigen Prozeßaktion, welche zue Steuerung eines Röntgenapparates in einer vorbestimmten Art führt.

Unter Berücksichtigung des Vorstehenden wird nunmehr auf die Fig. 4 Bezug genommen, wo ein Blockdiagramm des erfindungsgemäßen Gegenstandes nach Fig. 1 detaliert dargestellt ist. Wie aus dieser Fig. hervorgeht, werden durch die Position 44 die drei Busse 28, 30 und 32 (in den Figuren 2 und 3 dargestellt) und digitale Bindeglieder zusammengefaßt dargestellt durch die der Rechner 12 mit dem Röntgengenerator an dem drei Röntgenröhren 46, 48 und 50 angeschlossen sind, über eine Anzahl von elektronischen Kopplungskreisen 14 verbunden ist, welche auf digitale Signale des Rechners 12 ansprechen und geeignet sind, die erforderlichen analogen Steuersignale hervorzurufen und zu reagieren mit analog und/oder digital sich darstellenden Rückführungssignalen des Antriebs- und Leistungsversorgungskreises 52. Dieser ist geeignet, Funktionen auszuführen, die in allen Röntgendiagnostiksystemen mit einer Anzahl von durch den Benutzer wählbaren Techniken oder Verfahren auftreten. Der elektronische Kopplungskreis weist z.B. einen Brennfleck-Wählkreis 54, einen Röntgenröhrenwählkreis 56, einen Röntgenröhrenspannungs-Wählkreis 58, einen Röntgenröhrenstromwähl- und Regelkreis 60, einen Drehanodengeschwindigkeitswähl- und Steuerkreis 62, einen Belichtungs-Zeitkreis 64, einen Abkühlungskontrollkreis 66 für die Röntgenröhre und einen eine Foto- oder Kinokamera auswählenden Kreis 68 auf. Da alle diese Schaltungen digitale Eingaben akzeptieren, sind sie mit dem Antriebs- und Leistungsversorgungskreis 52 durch geeignete digital/analog oder analog/digital-Wandlerkreise 70 verbunden.

Zusätzlich zu den digitalen Steuersignalen, die den Kreisen 54 bis 68 von dem Mikroprozessor 12 geliefert werden, sind ein oder mehrere andere Rückführungssignale wie z.B. der an einem Widerstand 72 Abgegriffene Röntgenröhrenstrom — zurückgeführt zu den Bussen 44 über z.B. ein Multiplex-Glied 74 und einen Analog/Digital-Wandler 76.

Zusätzlich werden andere Signale — wie z.B. die Anodentemperatur und die von der Röntgenröhre 50 und dem zugeordneten Kollimator 78 gebildete Feldgröße — ebenfalls zurückgeführt, und zwar z.B. über das Multiplex-Glied 74 und den A/D-Wandler 76. Ebenfalls ist in Fig. 4 schematisch ein photoelektrischer Abtaster 80 vorgesehen, der am Patientenlagerungstisch 82, und zwar in der Nähe des Bildempfängers 84 angeordnet ist. Das Signal P.T. von der Einrichtung 80 und das den Abstand zwischen der Strahlenquelle und der Bildschicht oder der Strahlenquelle und dem Objekt repräsentierende Signal SID der Vorrichtung 86, sind ebenfalls zurückgeführt zu den Bussen 44 über z.B. das Multiplex-Glied 74 und den Analog/Digital-Wandler 76.

Es ist also aus Fig. 4 zu erkennen, daß die digitale Steuerung des Röntgenapparates aus verschiedenen elektronischen Kopplungskreisen 14 gebildet ist und daß bestimmte Rückführungssignale entlang der Adress-Daten- und Kontroll-Busse in Übereinstimmung mit einem in dem digitalen Informationsspeicher 34 nach Fig. 2 gespeicherten Programm zu dem Mikroprozessor 12 zurückgeführt sind.

Die Fig. 5—11 stellen in Flußdiagrammform eine Mehrzahl von Algorithmen für die gespeicherten Programmoperationen der Röntgeneinrichtung nach Fig. 4 dar. Die durch den Algorithmus vorgegebene Operation ist in dem Mikroprozessor 12 bewerkstelligt, und zwar in Abhängigkeit von den vorher in das PROM 34A (Fig. 2) einprogrammierten Instruktionen. Jeder Schritt oder jede Entscheidung, die in diesem Flußdiagramm angegeben ist, kann in aktueller Ausführung einen oder mehrere Schritte oder Entscheidungen umfassen, wobei jeder Schritt gegebenenfalls ein vollständiges Unterprogramm darstellt. Um jeden Schritt oder jede Entscheidung zu verwirklichen, ist es insbesondere dort, wo mechanische Abläufe erforderlich sind, notwendig, daß eine Zeit verstreicht, bevor andere Schritte durchgeführt werden können. Demgemäß müssen Verzögerungen in die Schritt für Schritt ablaufende, durch den Algorithmus angezeigte Operation eingefügt sein.

In dem Flußdiagramm nach Fig. 5 sind nunmehr die Verfahrensschritte in einem Algorithmus zur Bewerkstelligung des Zieles der vorliegenden Erfindung dargestellt, d.h. es ist hierin festgehalten, wie bei der Herstellung einer Röntgenaufnahme die Bildqualität insbesondere die Auflösung optimiert und gleichzeitig die Lebensdauer der Röntgenröhre maximiert werden kann. Der in Fig. 5 dargestellte Algorithmus basiert auf den zuvor erwähnten drei Zielen:

1) der gewüngschte Einsatz der Röntgenröhre unter Brennfleck- und Anodengeschwindigkeits-Bedingungen nach einer vorbestimmten Rangordnung,

2) die gewünschte vollständige Konstanthaltung des Filmschwärzungsfaktors während der Veränderung der Belichtungsparameter, Röntgenröhrenspannung, Röntgenröhrenstrom und Belichtungszeit nach einer vorbestimmten Art, und zwar bis eine geeignete, zulässige Röntgenröhren Belastungsdiagramm-Bedingung gefunden ist und eine Aufnahme gemacht werden kann,

3) der Gebrauch der minimal möglichen Aufnahmezeit, und zwar um die Verwischungseffekte infolge Bewegung des Objektes zu eliminieren.

Das Flußdiagramm nach Fig. 5 geht von einer Startstufe 100 aus, der eine Stufe 102 nachgeordnet ist, und zwar für die Eingabe der von dem Benutzer direkt oder organprogammiert gewählten Belichtungsparameter in den Mikroprozessor. Der Prozessor veranlaßt eine Entscheidungsstufe 104 festzulegen, ob der Wert der mAs, d.h. das Produkt des eingegebenen Röntgenröhrenstromes mal der Belichtungszeit eingegeben und vorhanden ist oder nicht. Wenn die Entscheidung positiv ist, wird die Stufe 106 angeregt. Wenn die Entscheidung dagegen negativ ist, wird eine Stufe 108 angeregt, von der die eingegebenen Werte des Röntgenröhrenstromes und der Belichtungszeit miteinander multipliziert werden, worauf dann die Stufe 106 angeregt wird. Die Stufe 106 bewirkt eine Berechnung, wobei das mAs-Produkt durch den maximalen, von dem Röntgengenerator vorgegebenen Röntgenröhrenstrom dividiert wird, so daß aus der Rechnung die minimal mögliche Belichtungszeit für das gewählte mAs-Produkt resultiert. Die Belichtungskurven der Röntgenröhren sind in das PROM 34A nach bekannter Art einprogrammiert, und zwar wie es z.B. in dem US-Patent 4 035 648 angegeben ist. Diese minimal mögliche Belichtungszeit zusammen mit dem maximalen Röntgenröhrenstrom und der eingegebenen Röntgenröhrenspannung, werden in der Stufe 110 verglichen und mit dem Belastungsdiagramm der ersten Priorität, d.h. z.B. kleiner Brennfleck und Standardgeschwindigkeit. Die Stufe 112 gibt an, ob das Belastungsdiagramm überschritten ist oder nicht. Falls dies nicht der Fall ist, so erlaubt eine Stufe 114 die Ausführung der Belichtung. Wenn jedoch das Belastungsdiagramm überschritten wird, d.h. die Entscheidung der Stufe 112 ist positiv, so wird ein Versuch gemacht, den Betrieb nach der ersten Priorität, d.h. kleiner Brennfleck und Standardgeschwindigkeit aufrechtzuerhalten, und zwar indem man eine Modifizierung der Belichtungsfaktoren nach zwei verschiedenen Verfahren durchführt. Bevor jedoch irgendwelche weiteren Schritte durchgeführt werden, wird durch die Entscheidungsstufe 116 festgelegt, ob die vorher errechnete Belichtungszeit kleiner ist als irgendein vorbestimmter Wert, d.h. die maximale Belichtungszeit, welche zulässig ist, um den Verwischungseinfluß so klein wie möglich zu halten. Diese vorbestimmte Zeit kann verschieden sein für unterschiedliche diagnostische Verfahren und verschiedene Patienten, und kann demgemäß durch den Benutzer einprogrammiert werden. Wenn das Ergebnis von der Entscheidungsstufe 116 angezeigt ist, daß die Zeit kleiner ist als diese maximale Belichtungszeit, wo wird eine Stufe 118 für eine Veränderung der Faktoren des mAs-Produktes wirksam, wobei aber das mAs-Produkt selbst konstant gehalten wird, und zwar veranlaßt der Prozessor eine Abnahme des Röntgenröhrenstromes und eine proportionale Zunahme der Belichtungszeit. Bei einem Röntgenapparat, bei dem der Röntgenröhrenstrom in mehreren bestimmten Stufen einstellbar ist, steuert der Prozessor diesen auf die nächst tiefere Stufe und dann wird zwecks Einhaltung eines konstanten mAs-Produktes die Steuerung auf eine entsprechend längere Belichtungszeit vorgenommen. Dies kann exakt durchgeführt werden, wenn die Zeit kontinuierlich einstellbar ist, wie es mit konventionellen digitalen Kreisen möglich ist. Ist jedoch die Zeit nur in bestimmten Stufen einstellbar, dann ist dieser Vorgang nur annähernd durchzuführen. Wenn jedoch die folgende spezielle Maßnahme bzgl. der Auswahl der bestimmten Stufen des Röntgenröhrenstromes und der bestimmten Stufen der Belichtungszeit durchgeführt wird, dann kann das mAs-Produkt konstant gehalten werden. Dies kann dadurch bewerkstelligt werden, daß die aufeinanderfolgenden Stufen des Röntgenröhrenstromes

voneinander durch einen feststehenden Prozentsatz %ΔmA getrennt sind, und daß die aufeinanderfolgenden Stufen der Belichtungszeit ebenfalls durch einen feststehenden %ΔT Prozentsatz voneinander getrennt sind, und dann die beiden Begriffe in eine integrale Beziehung zu setzen, und zwar

$$\frac{(\%\Delta mA)}{(\%\Delta T)} = N,$$

wobei N eine ganze Zahl, z.B. 1, 2, 3, . . . ist.

Nach der vorstehend beschriebenen Veränderung dieser beiden Belichtungsfaktoren erfolgt eine Rückkehr zu den Stufen 110 und 112, und zwar um festzustellen, ob die Belichtung mit diesen Parametern nach der Art der ersten Priorität möglich ist, d.h. kleiner Brennfleck und Standardgeschwindigkeit. Wenn dies noch nicht möglich ist, wird wiederum eine Absenkung des Röntgenröhrenstromes bei Stufe 118 durchgeführt, und zwar solange wie eine vorbestimmte maximale Grenze für die Belichtungszeit bezogen auf den Verwischungseffekt nicht überschritten wird. Sobald diese maximale Belichtungsgrenze erreicht ist, wird die Zurücknahme des Röntgenröhrenstromes über Stufe 118 fallengelassen. Anstatt dessen wird eine zweite Art der Veränderung der Belichtungsfaktoren über die Stufe 120 durchgeführt, und zwar wird dabei der Röntgenröhrenstrom auf den nächst niedrigeren einstellbaren Wert verändert, während die Belichtungszeit konstant gehalten wird, weil sie die Zeitgrenze bzgl. des Verwischungseffektes erreicht hat. Um jedoch den Schwärzungsfaktor unverändert aufrechtzuerhalten, nimmt die Röntgenröhrenspannung gemäß der Entscheidungsstufe 122 zu. Die Röntgenröhrenspannung nimmt dabei proportional zu dem Röntgenröhrenstrom derart zu, daß der Filmschwärzungsfaktor konstant gehalten wird. Wenn dieser Belichtungsfaktor innerhalb des Röntgenröhren-Belastungsdiagramms liegt, dann kann eine Belichtung in Übereinstimmung mit den Stufen 110, 112 und 114 angefertigt werden. Ansonsten wird der ganze Prozeß wiederholt, und zwar bis die Entscheidungsstufe 122 anzeigt, daß die Grenze für die mögliche Röntgenröhrenspannungserhöhung für das Röntgenröhren-Belastungsdiagramm der ersten Priorität erreicht ist. In diesem Punkt ist also durch das Programm festgelegt, daß eine Belichtung mit dem Belastungsdiagramm der ersten Priorität nicht möglich ist, sodaß ein Übergang zu dem nächst wünschenswerten Belastungsdiagramm, d.h. kleiner Brennfleck, Maximalgeschwindigkeit erfolgen muß, was durch die Stufe 124 eingeleitet wird. Der Prozeß wird wiederholt, und zwar solange, bis alle Röntgenröhren-Belastungsdiagramme, ausgehend von den am meisten wünschenswerten Belastungsdiagrammen, d.h. großer Brennfleck und Maximalgeschwindigkeit, untersucht worden sind. Wenn alle Belastungsdiagramme untersucht worden sind und eine Belichtung nicht möglich ist, wird über die Entscheidungsstufe 126 die Stufe 128 angeregt, die eine Anzeige und/oder eine Verhinderung weiterer Operationen des Systems vornimmt, und zwar solange, bis ein neuer Satz von Belichtungsfaktoren in die Einrichtung über die Stufe 102 eingegeben worden ist.

Falls gewünscht, ist aber eine bewußte Überschreitung der Maßnahmen durch den Benutzer in Notfällen erlaubt.

Sobald ein vom Benutzer ausgewählter Satz von Belichtungsfaktoren in das System eingegeben worden ist, beginnt das Programm — wie in Fig. 5 angezeigt — den größten Wert des Röntgenröhrenstromes herzustellen, der noch für Röntgengeneratoren zulässig ist und dann die Berechnung des korrespondierenden Wertes der minimal möglichen Belichtungszeit vorzunehmen. Diese Werte werden dann mit dem Belastungsdiagramm der ersten Priorität vergleichen. Wenn die Faktoren mit dem Belastungsdiagramm übereinstimmen, unternimmt es die notwendigen Schritte, um eine Belichtung zu ermöglichen. Wenn das Belastungsdiagramm überschritten wird, ruft das Programm eine Modifizierung des zuerst angewandten Satzes von Belichtungsfaktoren derart vor, daß, wenn es die berechnete minimale Belichtungszeit, die mit dem maximalen Röntgenröhrenstrom korrespondiert, kleiner ist als irgendein maximal zulässiger, in dem Programm zwecks Verhinderung des Verwischungseffektes gespeicherter (oder durch den Benutzer eingegebener) Wert, ein Wechsel der Belichtungsfaktoren in solch einer Weise durchgeführt wird, daß das mAs-Produkt konstant bleibt, und zwar durch Reduzierung des Röntgenröhrenstromes und Erhöhung der Belichtungszeit um einen vorgeschriebenen Betrag. Die neuen, veränderten Belichtungsfaktoren des Röntgenröhrenstromes und der Belichtungszeit werden nun mit dem Belastungsdiagramm verglichen, und zwar in der Hinsicht, ob nun eine Belichtung vorgenommen werden kann oder nicht. Wenn eine Belichtung immer noch nicht durchgeführt werden kann, und zwar, weil das Belastungsdiagramm überschritten wird, wird eine weitere Reduzierung des Röntgenröhrenstromes und eine korrespondierende Erhöhung der Belichtungszeit versucht, und zwar soweit die maximale Belichtungszeit noch nicht überschritten ist. Nach mehrfachen Wechseln bei gleichzeitiger Aufrechterhaltung eines konstanten mAs Produktes wird eine andere Methode herangezogen, und zwar, wenn die maximale Zeit bezogen auf den Verwischungseffekt erreicht ist. Dabei wird eine Reduktion des Röntgenröhrenstromes und eine Erhöhung der Röntgenröhrenspannung durchgeführt, und zwar bei gleichzeitiger Konstanthaltung des Filmschwärzungsfaktors. Wenn die Grenze der Röntgenröhrenspannungsveränderung noch nicht überschritten ist, führt das Programm eine solche Änderung durch Reduzierung des Röntgenröhrenstromes und Erhöhung der Röntgenröhrenspannung durch, wobei der neue Satz von Parametern wiederum mit dem Belastungsdiagramm verglichen wird.

Wenn eine Kombination von "Konstant mAs-Wechseln" und "Röntgenröhrenspannungswechseln" nicht dem Satz von Faktoren entspricht, welcher eine Belichtung ermöglicht, dann ist eine Belichtung mit dem Belastungsdiagramm der ersten Priorität nicht möglich, was zu einem Übergang zu dem nächst wünschenswerten Belastungsdiagramm führt, wobei der Prozeß wiederholt und mit den von dem Benutzer eingegebenen Originalfaktoren begonnen wird, und zwar in der Hoffnung, daß eine Belichtung nunmehr durchgeführt werden kann. Nacheinander werden dann alle möglichen Belastungsdiagramme in der Reihenfolge ihrer Rangfolge durchgegangen, und zwar bis entweder ein zulässiger Satz von Belichtungsfaktoren ermittelt worden ist, oder es sich herausgestellt hat, daß auf Grund der Zwänge des Programms eine Belichtung nicht möglich ist.

Die durch den Mikroprozessor gesteuerte Röntgeneinrichtung gemäß der Erfindung, ist geeignet, zusätzliche Funktionen zu übernehmen wie z.B. Überprüfung der vorhandenen Wärmekapazität der Drehanode. Solch ein Algorithmus ist für den Fall von einer einzigen Belichtung in Fig. 6 dargestellt.

Aus Darstellungsgründen ist angenommen, daß diese Maßnahme eingebettet ist in das Operationssystem, welches Belichtungen gestattet, außer, wenn sie verboten sind. Das Operationssystem kann derart sein, daß zu jeder Zeit neue Belichtungsfaktoren eingegeben werden können, wenn diese Maßnahme abgerufen wird. Falls neue Belichtungsfaktoren eingegeben worden sind, und zwar über die Startstufe 200, wird die Belichtung zuerst von der Stufe 201 verhindert. In der Stufe 202 wird die Energie der durchzuführenden Belichtung berechnet und in der Stufe 204 zu der bereits in der Drehanode vorhandenen Wärmeenergie addiert. Eine nachfolgende Entscheidungsstufe 206 bestimmt, ob die Wärmekapazität der Drehanode überschritten wird oder nicht. Wenn dies nicht der Fall ist, läßt die Stufe 208 die Belichtung zu, und zwar durch Aufhebung des Verbotes und das Programm geht weiter zu zwei nachfolgenden Entscheidungsstufen 210 und 212, welche ermitteln, ob neue Faktoren eingegeben worden sind oder nicht, und ob die durchzuführende Belichtung bereits gemacht worden ist oder nicht. Wenn die Antwort auf diese beiden Forderungen negativ ist, wird die Stufe 208 wiederum angeregt, und zwar um die Belichtung zu ermöglichen. Wenn eine der von den Stufen 210 und 212 gegebenen Antworten positiv ist, kehrt das Programm zu der Stufe 201 zurück. Es ist zu bemerken, daß eine Belichtung nunmehr nicht erlaubt ist, und zwar, weil sie außerhalb des erlaubten Rahmens liegt. Wenn die Stufe 206 eine positive Entscheidung gibt, d.h. daß die Wärmekapazität überschritten ist, dann wird die Rechenstufe 214 angeregt, worin die Zeit für die zwecks durchzuführender Belichtung notwendige Abkühlung ermittelt wird. Die Pausenzeit wird dann an der Stufe 216 angezeigt. Es ist zu bemerken, daß bei diesem Punkt das Belichtungsverbot der Stufe 201 noch nicht aufgehoben worden ist. Eine Entscheidungsstufe 218 ist der Stufe 216 nachgeschaltet, wodurch, wenn neue Faktoren eingegeben worden sind, das Programm zu der Stufe 201 zurückkehrt. Wenn aber andererseits eine negative Entscheidung bzgl. der Eingabe von neuen Faktoren von dieser Entscheidungsstufe gegeben wird, werden die Schritte 206, 214 und 216 wiederholt, und zwar solange, bis solche neuen Belichtungsfaktoren in das System eingegeben worden sind, oder die Entscheidungsstufe 206 negative geworden ist, d.h. daß angezeigt wird, daß die Drehanode sich genug abgekühlt hat, um eine Belichtung zu erlauben. Der Wert der in der Drehanode gespeicherten Wärmeenergie kann durch einen digitalen oder einen analogen Schaltkreis simuliert werden oder durch ein in dem Prozessor selbst vorhandenes Unterprogramm. Mehrere alternierende Anzeigen sind ebenfalls möglich, so z.B. die Anzahl der Belichtungen einer durchzuführenden Art, welche berechnet sein kann, oder die Anzahl von erlaubten Sekunden der Laufzeit für eine spezielle Filmgeschwindigkeit von einer Fotokamera, oder die Anzahl von Sekunden der Laufzeit einer Kinokamera, die in Übereinstimmung mit der gespeicherten Wärmeenergie der Drehanode und der Wärme kapazität der Röntgenröhre vergehen kann.

Irgendeine oder alle diese Berechnungen können in der Stufe 214 durchgeführt und die Ergebnisse durch die Stufe 216 angezeigt werden.

In Fig. 7 ist der Algorithmus für die Berechnung der notwendigen Pausenzeiten zwischen aufeinanderfolgenden Belichtungen bei Tomographieserien dargestellt. Basierend auf den in den Röntgendiagnostikapparat eingegebenen Belichtungsfaktoren nimmt der Mikroprozessor die Berechnung der für jede Belichtung und die Anzahl der geplanten Belichtungen notwendigen Pausenzeiten zwischen aufeinanderfolgenden Belichtungen und falls erwünscht, die automatische Steuerung des Ablaufs der aufeinanderfolgenden Belichtungen vor. In Fig. 7 ist einer Startstufe 300 eine Rechenstufe 302 nachgeordnet in der die Multiplikation von N × kV × mA × T durchgeführt wird, wobei N die Anzahl der Filme von einer Serie und T die Belichtung eines Films darstellt. Die Pausenzeit $T_P$ wird dann mit Stufe 304 berechnet, und zwar nach der Beziehung

$$T_P = \frac{E-(H_c-H_i)}{K(N-1)} - \frac{(N)}{N-1} \times T_e$$

wobei E die gesamte Energie der Serien darstellt und aus der Berechung in Stufe 302 resultiert. $H_c$ stellt dabei die Wärmekapazität des Gehäuses dar. $H_i$ stellt dabei den vorliegenden Wert der gespeicherten Wärmeenergie dar, die kontinuierlich durch andere Mittel korrigiert wird, wie z.B. einen analogen Simulator für die in der Drehanode gespeicherte Wärmeenergie oder einem anderen, in dem Informationsspeicher des Mikroprozessors vorhandenen Programm, wobei jede durchgeführte Belichtung und die folgende Abkühlung der Röntgenröhre berücksichtigt wird. Schließlich stellt K dabei die Abkühlungsrate des Gehäuses und $T_e$ die tomographische Ablaufzeit oder die Belichtungszeit dar.

Der Stufe 304 ist eine Entscheidungsstufe 306 nachgeordnet, und zwar zur Bestimmung, ob die Pausenzeit $T_P$ größer ist als die notwendige minimale Pausenzeit $T_{min}$. Diese minimale Pausenzeit ist erforderlich für die Zwecke, die sich auf das Tomographieverfahren selbst beziehen z.B. auf die mechanische Rückstellung der Scann-Apparatur oder die Ermöglichung der Atmung bzw. der momentanen Entspannung des Patienten usw.

Wenn die Entscheidung negativ ist, setzt die Stufe 308 die Pausenzeit auf das Minimum und macht darüber eine Anzeige. Wenn die Entscheidung der Stufe 306 dagegen positiv ist, gibt die Stufe 310 den Befehl zur Ermöglichung der Belichtung und gleichzeitige Anzeige der Pausenzeit $T_p$, und wenn erwünscht, der gesamten Serienzeit $T_s$, wobei die Serienzeit $T_s$ gleich der Bezeichnung $N \times T_e + (N - 1)T_p$ ist. Im Anschluß an die Stufe 310 wird der Prozeß wiederholt, wenn neue Faktoren vorliegen, und zwar beginnend mit der Stufe 302, wodurch eine Neuberechnung erfolgt und die Wärmeberechnungen auf den neuesten Stand gebracht werden.

In Fig. 8 ist ein Fortsetzungsprogramm angegeben, welches geeignet ist, die Röntgenröhren-Historie zu zpeichern. Diese stellt eine Registrierung des Gebrauchs der Röntgenröhre(n) in Bezug auf die Anzahl der Belichtungen, den Betrag der Drehanodenbelastungen und die Anzahl von Belichtungen unter verschiedenen Einstellbedingungen dar. Ausgehend von der Stufe 400 beginnt das Flußdiagramm nach Fig. 8 mit einer Entsheidungsstufe 402 zur Feststellung ob ein Verlangen für einen Ausdruck der Röntgenröhren-Historie besteht. Wenn die Antwort positiv ist, wird die Stufe 404 wirksam, die einen entsprechenden Ablauf einleitet. Wenn die Antwort negativ ist, wird eine zweite Entscheidungsstufe 406 angeregt, durch die ermittelt wird, ob es eine unwirksame Belichtung gegeben hat oder nicht. Wenn die Antwort darauf positiv ist, läuft eine Serie von Instruktionen ab, die z.B. mit der Stufe 408 beginnt, die das Einholen der Belichtungsdaten wie z.B. der Röntgenröhrenspannung, des Röntgenröhrenstromes, der Belichtungszeit, des Brennflecks, der Anodendrehgeschwindigkeit und der Röntgenröhrennummer veranlaßt.

Auf diese Stufe folgt eine Stufe 410, durch die die gesamte Anzahl der gezählten Belichtungen der verwendeten Röntgenröhre erhöht wird. Dieser Stufe folgt dann noch eine Stufe 412, durch die die Anzahl der gezählten Belichtungen für diese Röhre bei spezifiziertem Brennfleck und Anodendrehgeschwindigkeit erhöht wird. Dieser Stufe ist dann eine Stufe 414 nachgeordnet, in welcher die Energie der Belichtung berechnet wird, und zwar durch Multiplizierung der Belichtungsfaktoren (Röntgenröhrenspannung, Röntgenröhrenstrom und Belichtungszeit). Nach Durchführung der Berechnung in Stufe 414 wird diese Belichtungsenergie zu der totalen Energie der betreffenden Röntgenröhre in einer Stufe 416 addiert und, wenn gewünscht, wird das Resultat an ein Kontrollprogramm für die Wärmeenergie der Drehanode gesandt, das nicht dargestellt ist.

Wie vorerwähnt, ist der erfindungsgemäße Gegenstand fähig mit organprogrammierten Faktoren zu arbeiten, wobei der Benutzer lediglich den zu untersuchenden Bereich auswählt und die Art der Untersuchung, sowie den Patienten selbst berücksichtigt.

Der Mikroprozessor 12 und insbesondere das PROM 34A ist fähig für oder leicht anpaßbar auf die Speicherung und den Abruf von Belichtungsfaktoren auf einer anatomischen Basis, was in Fig. 9 dargestellt ist. Dieses Programm erfordert eine Eingabe durch den Benutzer mit Hilfe von einem Satz von Druckknöpfen, die z.B. den automatischen Bereich, die Technik, den gewünschten Blickwinkel, die Patientendicke und andere Faktoren wie z.B. das Alter spezifizieren. Diese Patienteninformation kann, wenn gewünscht, von einem externen Hospitalinformationssystem eingegeben werden. Der Startstufe 500 ist dabei die Stufe 502 nachgeordnet, welche anzeigt, daß der anatomische Bereich und die Technikwahl eingegeben sind, und welche eine Stufe 504 anregt, die gespeicherten Werte der richtigen Röntgenröhrenspannung und des mAs-Produktes auszugeben. Desweiteren werden Faktoren — wie z.B. die Dicke, das Alter usw. — über eine Stufe 506 eingegeben. In Abhängigkeit von diesen Faktoren korrigiert eine Stufe 508 die Röntgenröhrenspannung und/oder das mAs-Produkt. Dies ist prinzipiell übereinstimmend mit den heutzutage praktizierten Maßnahmen, wo drei Patientenwerte, und zwar dünn, mittel und dick betrachtet werden, und demgemäß die Röntgenröhrenspannung von einem mittleren feststehenden Wert verändert wird, und zwar nach oben, wenn der Patient dick ist und nach unten, wenn er dünn ist. Mit einem PROM kann ein viel komplexerer Algorithmus für einen solchen Ausgleich durchgeführt werden.

In einer Stufe 510 sind dann deshalb Daten für statistische Zwecke gespeichert. Dieser Stufe folgt eine Stufe 512, welche der Stufe 102 in Fig. 5 korrigierte Röntgenröhrenspannungs- und mAs Produkte sendet.

In Fig. 10 ist ein automatisiertes Operationsprogramm für das System nach Fig. 4 wiedergegeben. Es beginnt mit einer Eingangsstufe 600, der eine Stufe 602 für die Eingabe der Parameter nachgeordnet ist, woraus zunächst in einer Stufe 604 die technischen Faktoren berechnet werden, was in Übereinstimmung mit den Arten der Programme nach Figuren 5 und 8 erfolgen kann. Der Stufe 604 ist eine Entscheidungsstufe 606 nachgeschaltet, in der ermittelt wird, ob eine Belichtung möglich ist oder nicht.

Die Stufe 606 ist sehr umfassend, d.h. sie beinhaltet eine Vielfalt von Sicherheitsprüfungen wie z.B. ein Programm zur Überwachung der Wärmeenergie in der Drehanode gemäß Fig. 6, die Berechnung von Tomographieserien gemäß Beispiel nach Fig. 7, und wenn z.B. ein Tomographieverfahren durchgeführt wird, auch die anderen erforderlichen Prüfungen, unter anderem die Kontrolle des Antriebskreises der Drehanode, und zwar um zu gewährleisten, daß die Drehanode ihre jeweilige Geschwindigkeit erreicht hat, Überwachung des Zustandes der Röhrenabkühlung (z.B. vorhandener Wasserdurchfluß) und Prüfung des Zustandes der Filmkamera (d.g., ob die Kassette mit dem Film versorgt ist oder der Antriebsmotor läuft). Dies alles wird durch Unterprogramme verwirklicht, und zwar ähnlich dem dargestellten oder in einigen Fällen durch Unterbrechungssignale für den Mikroprozessor, welche den Grund für die Unterbrechung anzeigen und die notwendigen Schritte veranlassen, um dem Benutzer anzuzeigen, den Grund für die Unterbrechung zu beseitigen. Wenn die Entscheidung negativ ist, zeigt das Programm diese Tatsache an und kehrt zurück zu der Stufe 602. Wenn jedoch die Belichtung für den gewählten und über Stufe 608 eingegebenen Satz von Belichtungsfaktoren durchführbar ist, so werden die richtigen Signale zu den Kopplungskreisen 14 nach Fig. 4 gesandt, worauf eine zweite Entscheidungsstufe 610 ermittelt, ob der Belichtungsknopf gedrückt worden ist oder nicht. Wenn darauf eine positive Antwort sich einstellt, wird eine Stufe 612 angeregt, die eine Steuerung der Belichtung vornimmt. Dieser Stufe 612 ist eine Stufe 614 nachgeordnet, welche die Faktoren der Röntgenröhren-Historie oder andere Faktoren wie z.B. ein in Fig. 11 zum Ausdruck kommendes Pseudodosisprogramm registriert. Wenn demgegenüber die Entscheidungsstufe 616 wiederum eine Frage gestellt, und zwar, ob irgendeiner der Faktoren gewechselt worden ist. Wenn die Antwort darauf positiv ist, erfolgt eine Rückkehr zur Stufe 604, während auf eine negative Antwort eine Rückkher zur Stufe 610 erfolgt.

In Fig. 11 ist schließlich ein Flußdiagramm für ein Programm dargestellt, wobei die Patientendosis der Röntgenstrahlenenergie auf Grund der Berechnung geschätzt wird, die auf bekannten und gemessenen Parametern basiert. Wenn ein Strahlungssensor, wie z.B. die Einrichtung 80 nach Fig. 4, verwendet wird, kann die Ermittlung durch teilweise oder vollständige Messung erfolgen. In Fig. 11 ist der Startstufe 700 eine Entscheidungsstufe 702 nachgeschaltet, die ermittelt, ob eine Belichtung erfolgt ist oder nicht. Wenn die Antwort darauf positiv ist, erfolgt über eine Stufe 704 die Eingabe der Belichtungsparameter (Röntgenröhrenspannung, Röntgenröhrenstrom und Belichtungszeit).

Geometrische Eingangsparameter werden dann über eine Stufe 706 eingegeben. Diese Parameter bestehen z.B. aus der Feldgröße, dem Fokus-Bildempfängerabstand bzw. dem Fokus Objektabstand. Dann werden in einer Stufe 708 Werte für die Identifikation des Patienten eingegeben, der eine Rechenstufe 710 nachgeschaltet ist, welche eine Berechnung der Pseudodosis vornimmt, die auf folgender Beziehung basiert:

$$\text{Pseudodosis} = \frac{A \cdot mA \cdot (\text{Feldgröße}) \cdot f(kV) \cdot \cos\theta}{(SOD)^2}$$

wobei f(kV) eine Funktion der Röntgenröhrenspannung darstellt, welche empirisch bestimmt werden kann, $\theta$ ist der Einfallswinkel des Röntgenstrahlenbündels, die Bezeichnung SOD stellt den Abstand zwischen der Strahlenquelle und dem Objekt dar, und A ist eine Konstante, die von verschiedenen physikalischen Parametern abhängt, wie z.B. dem Alter der Röntgenröhre, das periodisch bestimmt und auf den neuesten Stand gebracht wird. Nach dieser Berechnung wird eine Stufe 712 angeregt, die eine Ausgabe des Resultates bewirkt oder das Resultat wird in anderen Registern gespeichert z.B. in der radiologischen Abteilung eines Systems wie es mit Nr. 22 in der Fig. 4 dargestellt ist.

Ein besonderer Vorteil des erfindungsgemäßen Gegenstandes ist noch darin zu sehen, daß er sehr wirtschaftlich ist.

**Patentansprüche**

1. Röntgeneinrichtung zur Erstellung radiologischer Abbildungen mit einer Eingabeeinheit (20) zur Wahl einer von mehreren vorgesehenen Röntgenröhren (46, 48, 50) mit einstellbarer Brennfleckgröße und Drehanodengeschwindigkeit, sowie zur Wahl der Werte des Röntgenröhrenstromes und der Belichtungszeit, und mit einer die Belastungskurven dieser Röntgenröhren für unter schiedliche Belichtungsparameter speichernden Datenverarbeitungseinheit (12), die die mit den gewählten Werten der Belichtungsparameter korrespondierende Röntgenröhrenspannungswerte anhand der zugehörigen Belastungskurve einstellt, dadurch gekennzeichnet, daß die Datenverarbeitungseinheit (12) für eine optimale Belichtung bei hinreichender Sicherheit vor Überlastung der Röntgenröhre im Rahmen eines Ablaufprogramms:

a) zunächst sowohl die Röntgenröhrenspannung als auch das Produkt aus Röntgenröhrenstrom und Belichtungszeit konstant hält, und den Röntgenröhrenstrom beginnend mit dem zulässigen Maximalwert solange erniedrigt, bis die zugehörige Belastungskurve eine Belichtung gestattet,

b) bei Nichtgewähren der Belichtung und Erreichen der maximal zulässigen Belichtungszeit die Röntgenröhrenspannung solange erhöht und den Röntgenröhrenstrom in der Abhängigkeit der Nebenbedingung konstanter Schwärzung solange erniedrigt, bis die zugehörige Belastungskurve eine Belichtung gestattet, und

c) die Belichtungsparameter zunächst unter Zugrundelegung der für die Bildauflösung optimalsten Belastungskurve des kleinsten Brennflecks und der Standardgeschwindigkeit der Drehanode ermittelt, und bei Nichtgewähren der Belichtung die gewählten Belichtungsparameter mit den für die Bildauflösung nächst optimalen Belastungskurven für unterschiedliche Werte des Brennflecks und der Anodendrehgeschwindigkeit vergleicht, und zwar zunächst mit der für den kleinsten Brennfleck und einer schnelleren Anodendrehgeschwindigkeit,
daß Mittel vorgesehen sind, die die gemäß dem Ablaufprogramm von der Datenverarbeitungseinheit (12) ermittelten Werte der Belichtungsparameter über entsprechende Wahlkreise (58 bzw. 60 oder 64) zu einem Antriebs- und Versorgunskreis (52) zur Einstellung des Hochspannungserzeugers weiterleiten.

2. Röntgeneinrichtung nach Anspruch 1, dadurch gekennzeichnet, daß die Datenverarbeitungseinheit die maximal zulässige Belichtungszeit in Abhängigkeit von der Bewegungsunschärfe des über die Eingabeeinheit (20) angewählten Organbereichs ermittelt.

3. Röntgeneinrichtung nach Anspruch 1 oder 2, dadurch gekennzeichnet, daß die Datenverarbeitungseinheit einen Mikroprozessor umfaßt, in dem neben dem Ablaufprogramm für die Optimierung der Belichtungsparameter noch weitere Operationsprogramme speicherbar sind.

4. Röntgeneinrichtung nach Anspruch 3, dadurch gekennzeichnet, daß wenigstens ein Operationsprogramm eine Operationsmenge aufweist, die die bei einer durchzuführenden Belichtung mit den vorgewählten Belichtungsdaten anfallende Wärmeenergie berechnet, und diese zu einem Wert addiert, der der in der in der Drehanode noch gespeicherten Wärmeenergie entspricht, und beim Überschreiten der Wärmekapazität der Drehanode die Belichtung verhindert.

5. Röntgeneinrichtung nach Anspruch 3 oder 4, dadurch gekennzeichnet, daß ein Operationsprogramm eine Operationsmenge aufweist, die die thermische Belastung der Röntgenröhre bei Tomographie-Serien in Abhangigkeit der beabsichtigten Belichtungen berechnet, und die die dabei zum Schutz der Röntgenröhre vor Überlast nötigen Pauseneinheiten zwischen den einzelnen Belichtungen ermittelt.

**Revendications**

1. Equipement radiologique pour la réalisation d'images radiographiques avec une unité d'entrée (20) pour la sélection d'un des tubes radiogènes (46, 48, 50) à foyer et vitesse d'anode tournante réglables, ainsi que pour la sélection des valeurs du courant du tube radiogène et du temps d'exposition, et avec une unité de traitement de données (12) stockant les courbes de charge de ces tubes radiogènes pour différents paramètres de charge et réglant les valeurs de tension du tube radiogène correspondant aux valeurs choisies pour les paramètres de charge en fonction des courbes de charge correspondantes, caractérisée par le fait que l'unité de traitement de données (12), assure une exposition optimale avec une sécurité suffisante contre la surcharge des tubes radiogènes et dans le cadre d'un programme de commande,

a) maintient tout d'abord constants à la fois la tension du tube radiogène et le produit du courant du tube radiogène et du temps d'exposition et réduit le courant du tube radiogène en commençant par la valeur maximale admissible jusqu'à ce que la courbe de charge correspondante autorise une exposition,

b) si l'exposition n'est pas autorisée et que le temps d'exposition maximale admissible est atteint, augmente la tension du tube radiogène et réduit le courant du tube radiogène en fonction de la condition secondaire d'un facteur de noircissement constant jusqu'à ce que la courbe de charge correspondante autorise une exposition, et

c) détermine les paramètres de charge tout d'abord en se fondant sur la courbe de charge optimale pour la résolution de l'image, à savoir celle d'un foyer minimal et d'une vitesse normale de l'anode tournante, et, si l'exposition n'est pas autorisée, compare les paramètres de charge sélectionnés avec les courbes de charge suivantes, dans l'ordre correspondant à une résolution optimale de l'image, pour différentes dimensions du foyer et de la vitesse de l'anode tournante, en commençant par la courbe pour le foyer le plus petit et une vitesse plus rapide de l'anode tournante,
et que des moyens sont prévus pour transmettre les valeurs des paramètres de charge déterminés suivant le programme de commande par l'unité de traitement de données (12), par des circuits de sélection appropriés (58 ou 60/64), à un circuit de commande et d'alimentation (52) assurant le réglage du générateur radiologique.

2. Equipement radiologique suivant la revendication 1, caractérisée en ce que l'unité de traitement de données détermine le temps d'exposition maximal admissible en fonction du flou de mouvement de la zone anatomique sélectionnée sur l'unité d'entrée (20).

3. Equipment radiologique suivant la revendication 1 ou 2, caractérisé en ce que l'unité de traitement de données comprend un microprocesseur dans lequel peuvent être stockés, outre le programme de commande pour l'optimisation des paramètres de charge, d'autres programmes opérationnels.

4. Equipment radiologique suivant la revendication 3, caractérisée en ce qu'au moins un programme opérationnel comprend une série d'opérations calculant l'énergie thermique correspondant à l'exposition à réaliser avec les facteurs de charge présélectionnés et ajoutant cette énergie à une valeur correspondant à l'énergie thermique encore stockée dans l'anode tournante, pour empêcher l'exposition en cas de dépassement de la capacité thermique de l'anode tournante.

5. Equipement radiologique selon la revendication 3 ou 4, caractérisée en ce qu'un programme opérationnel comprend une série d'opérations calculant la charge thermique des tubes radiogènes pour les série tomographiques, en fonction des expositions prévues, et déterminant les temps de pause nécessaires entre les différentes expositions afin de protéger les tubes à rayons X de la surcharge.

## Claims

1. X-Ray equipment designed for the realization of radiological imaging with an input unit (20) allowing the selection of one of several assigned X-ray tubes (46, 48, 50) with adjustable focus size and rotating anode speed as well as for the selection of values regarding the X-ray tube current and the exposure time and in conjunction with a data processing unit (12) storing the tube rating charts of these X-ray tubes for different exposure parameters and setting by means of the associating tube rating charts the tube tension values corresponding to the selected values of the exposure parameters, characterized owing to the fact that the data processing unit (12) for an optimum exposure with sufficient protection against overload of the X-ray tube within the scope of the basic routine:

a) maintains, first of all, both the tension of the X-ray tube and the product out of tube current and exposure time and decreases the tube current beginning with the permissible maximum value until the associating tube rating chart allows an exposure,

b) increases with non-granting of the exposure an attaining of the maximum permissible exposure time the tube tension and decreases the tube current in the dependence on the secondary condition of constant blackening until the associating tube rating chart allows an exposure and

c) determines the exposure parameters, first of all, taking as a basis for image resolution the optimum rating chart of the smallest focus and the standard speed of the rotating anode and with non-granting of the exposure compares the selected exposure parameters with the next optimum rating charts for image resolution for different values of the focus and the anode rotating speed and that, first of all, with that for the smallest focus and a faster anode rotating speed,

that means are provided that transmit in order the values of the exposure parameters determined as a result of the basic routine of the data processing unit (12) to set the high-tension generator by using corresponding selection circuits (58 resp. 60 or 64) to an energizing and supplying circuit (52).

2. X-Ray equipment according to Claim 1, characterized owing to the fact that the data processing unit determines the maximum permissible exposure time in dependence on the motional unsharpness of the organ area selected by the input unit (20).

3. X-Ray equipment according to Claim 1 or 2, characterized owing to the fact that the data processing unit contains a microprocessor that allows in addition to the basic routine for the optimization of the exposure parameters the storage of even further operation programmes.

4. X-Ray equipment according to Claim 3, characterized owing to the fact that at least one operation programme features a sequence of operations which computes with an exposure that has to be effected by using the preselected exposure data the accumulating thermal energy and adds this to the value that corresponds to the thermal energy still stored in the rotating anode and which inhibits the exposure in case the thermal capacity of the rotating anode is exceeded.

5. X-Ray equipment according to Claim 3 or 4, characterized owing to the fact that one operation programme features a sequence of operations which computes the thermal load of the X-ray tube with tomography-series in dependence on the intended exposure and in doing so determines in order to protect the X-ray tube against overload the necessary pauses between each exposure.

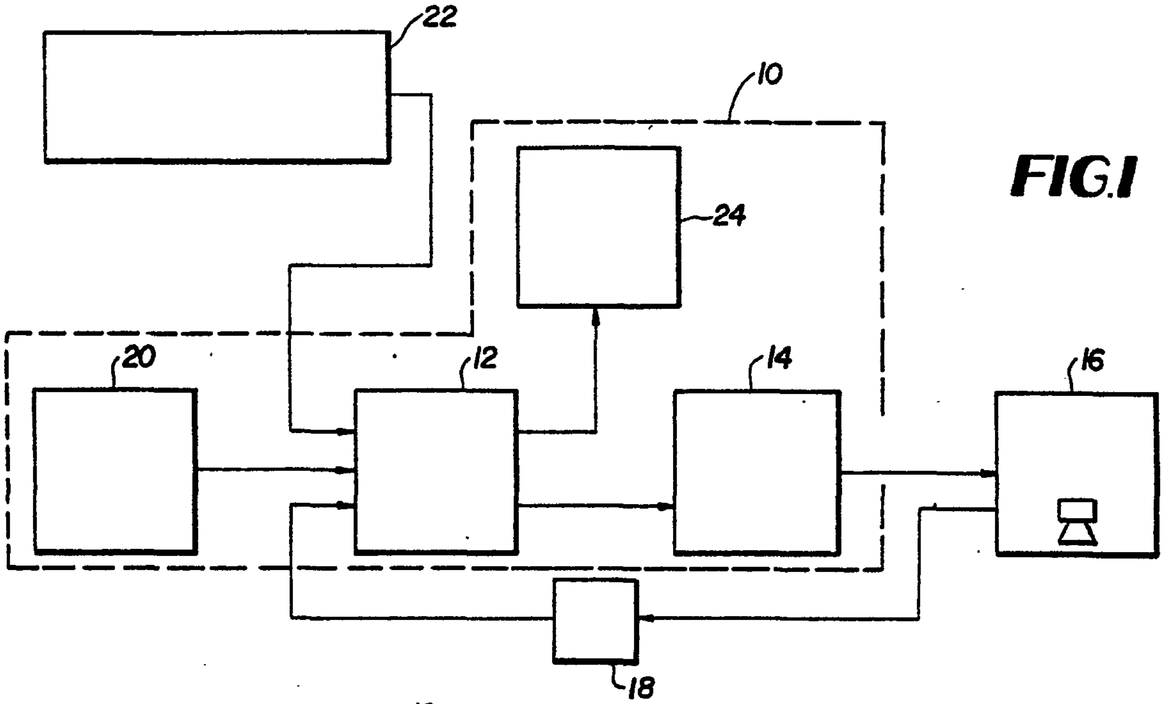


FIG.1

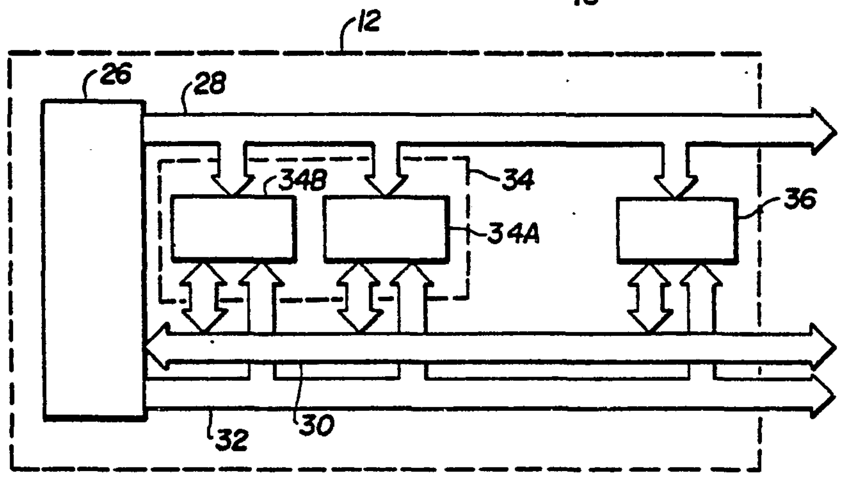


FIG.2

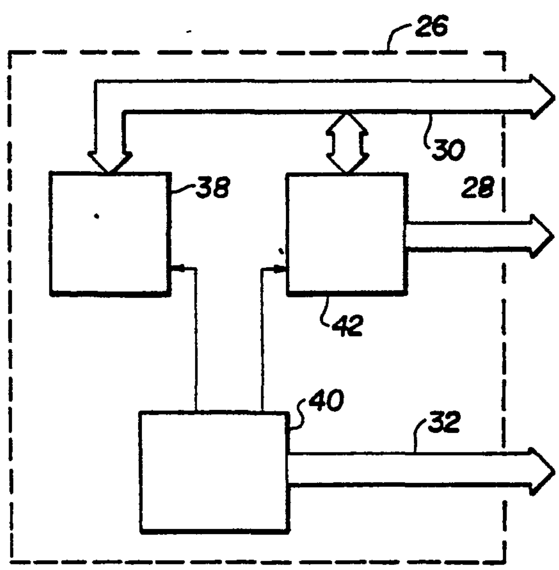


FIG.3

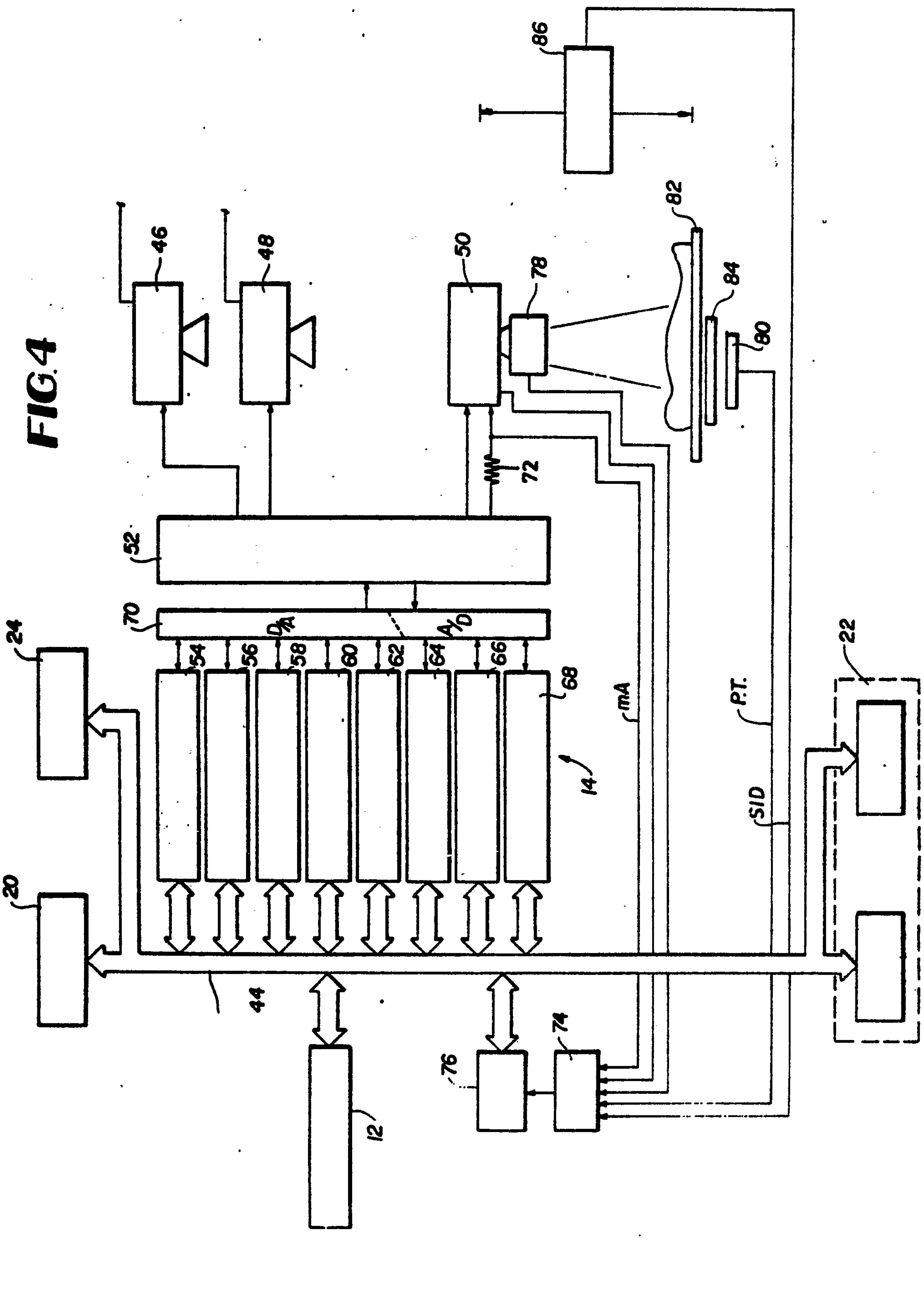

12
14
20
22
24
44
46
48
50
52
54
56
58
60
62
64
66
68
70
72
74
76
78
80
82
84
86
D/A
A/D
mA
P.T.
SID


FIG.4

FIG.5

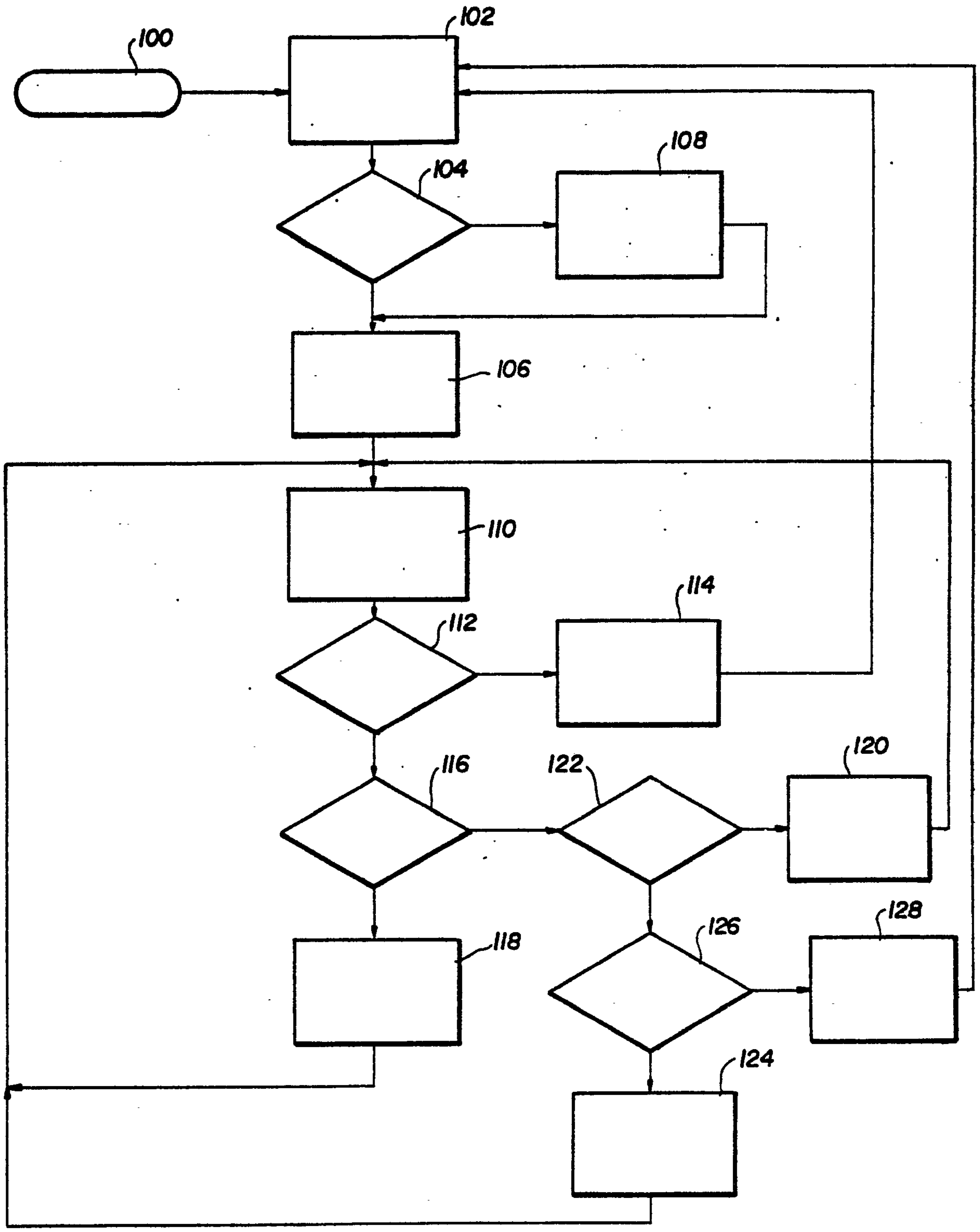

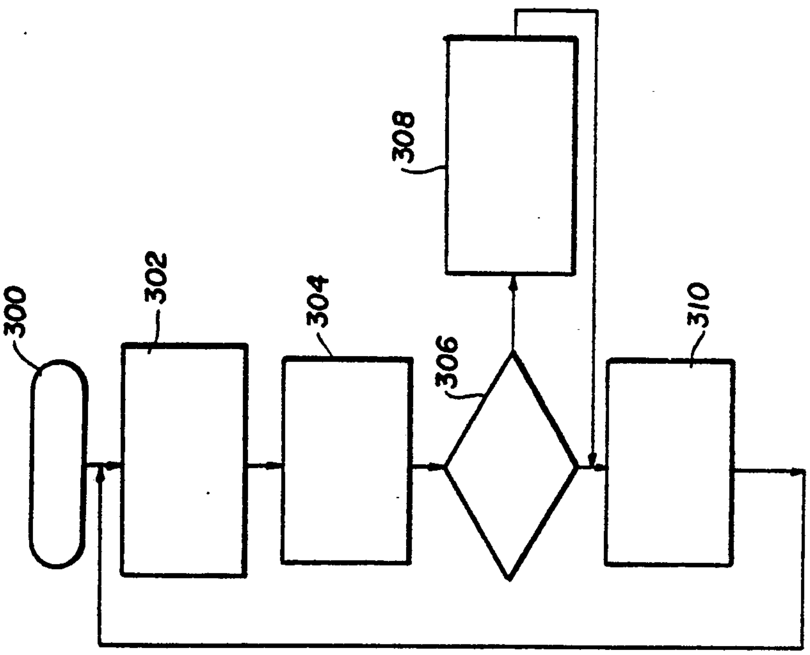
300
302
304
306
308
310

FIG.7

FIG.6

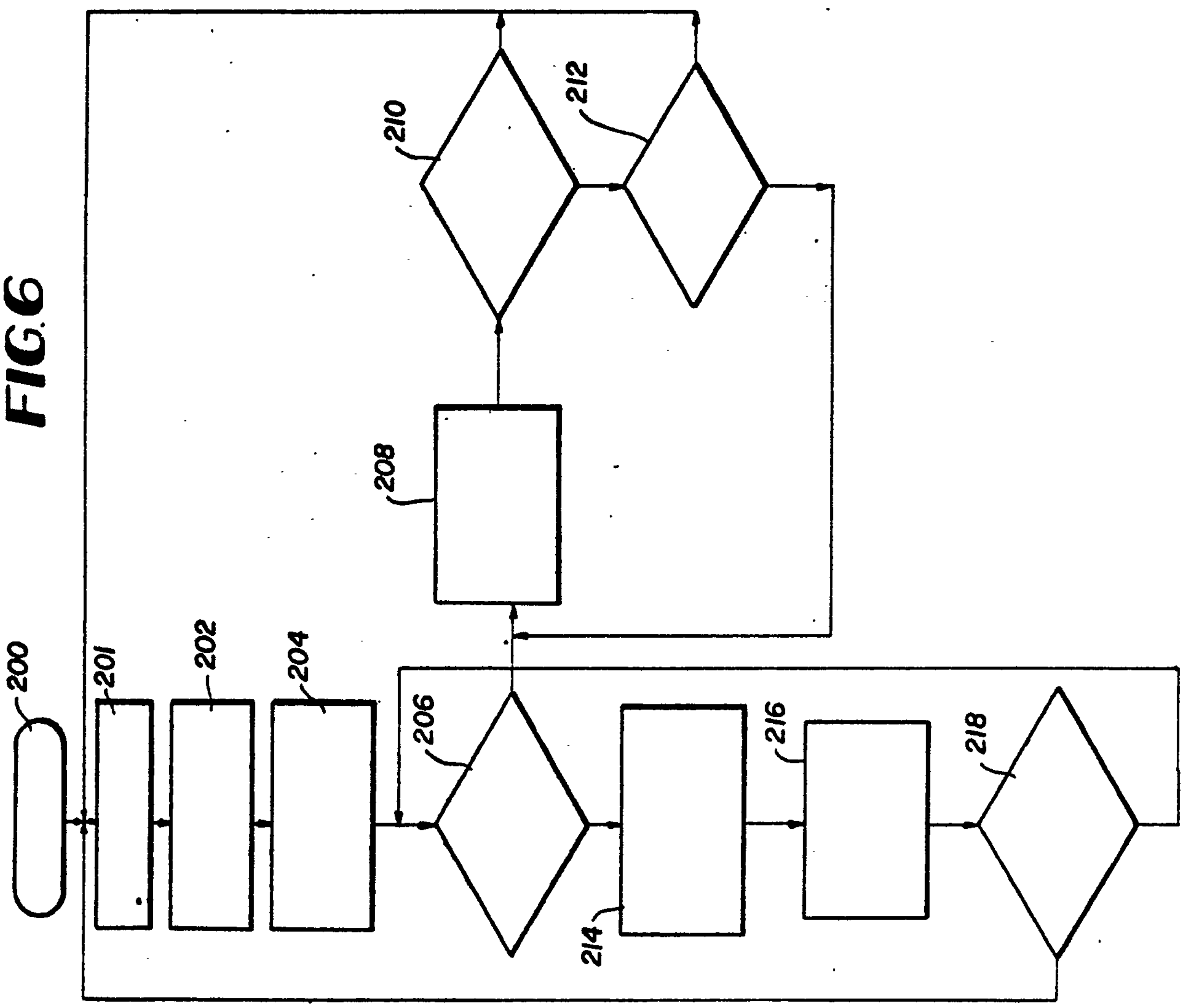
200
201
202
204
206
208
210
212
214
216
218

FIG. 8

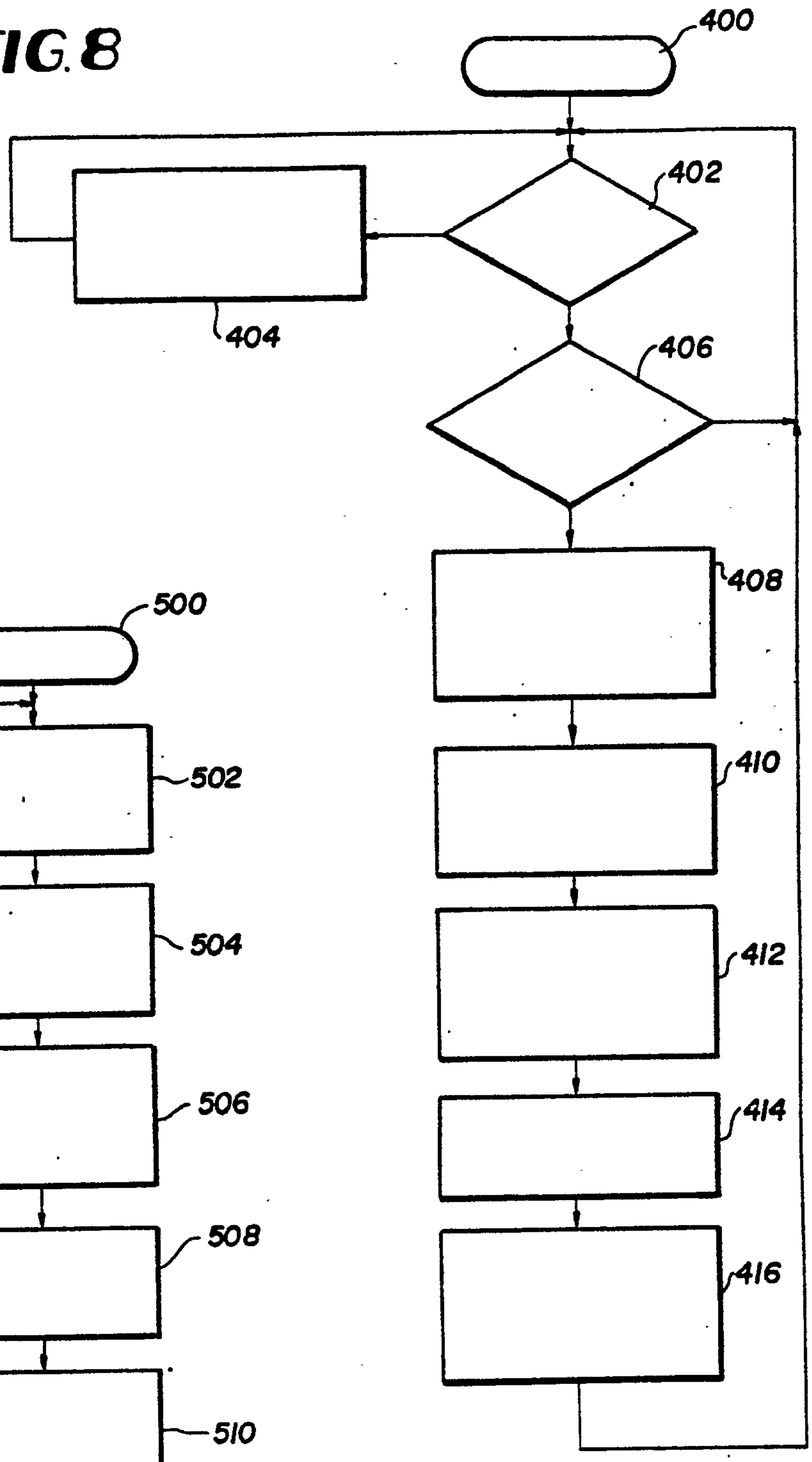


FIG. 9

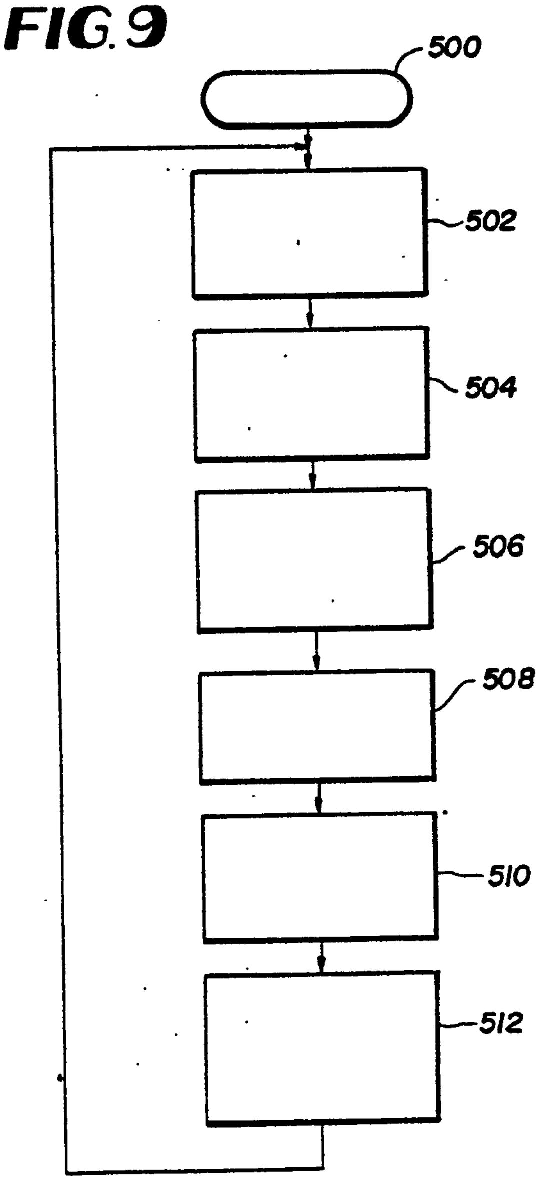

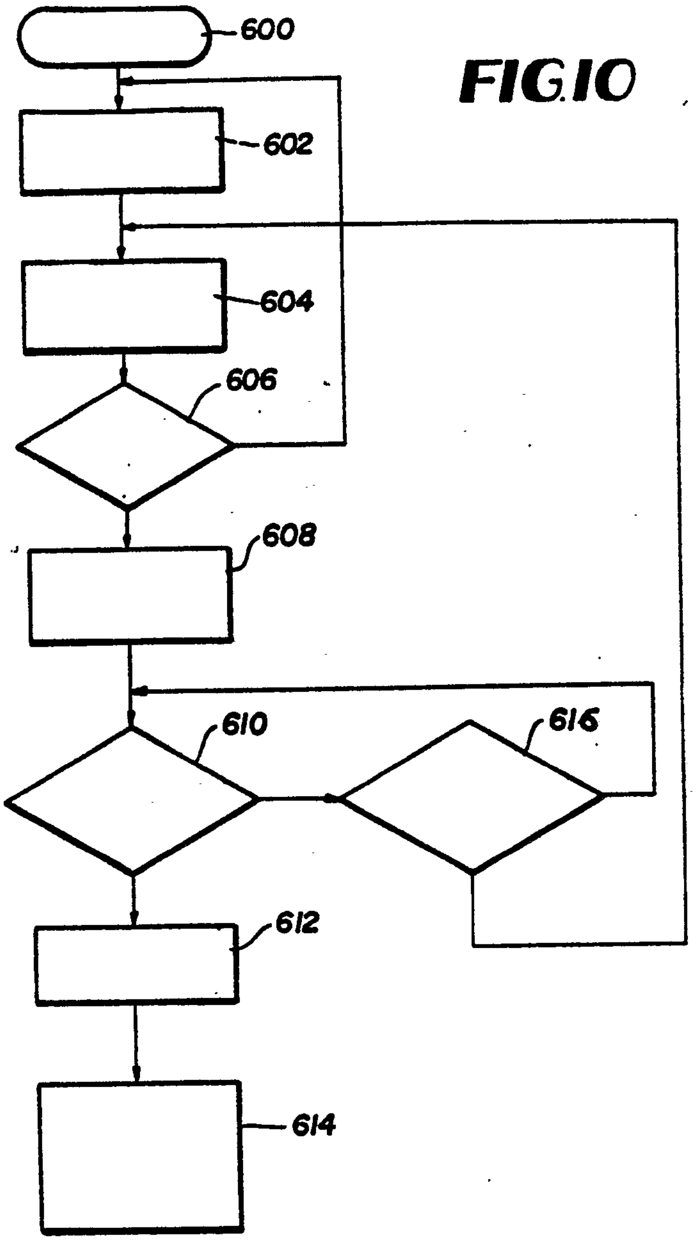


FIG.10

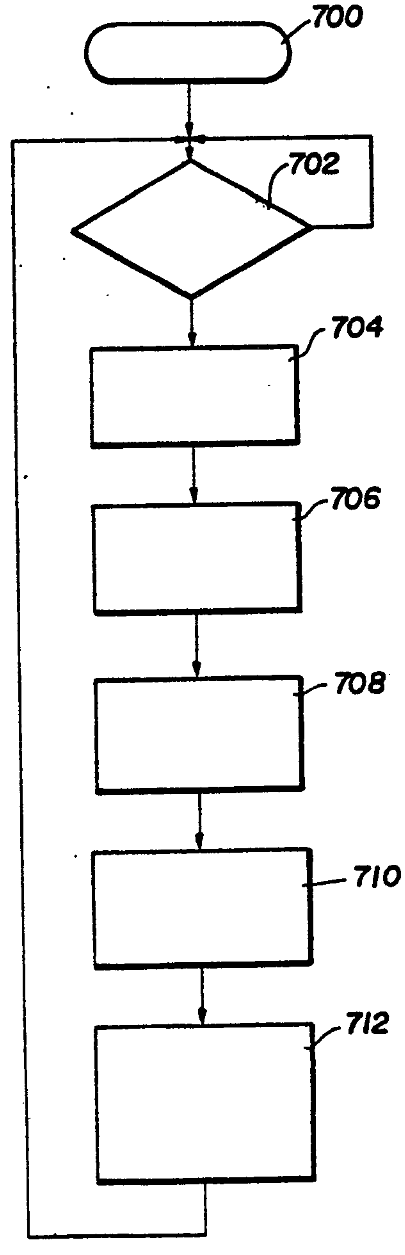


FIG.11